# EUROPEAN PATENT APPLICATION

(11) **EP 2 186 878 A1**
(43) Date of publication of application: **19.05.2010**
(21) Application number: 09175708.8
(22) Date of filing: 11.11.2009
(51) Int. Cl.: C12M 1/30, A61B 10/00, A61B 10/02

(54) **Coding for a sample carrier**

(30) Priority: 12.11.2008 DE 202008014965 U
(71) Applicant: EPPENDORF AG, 22339 Hamburg (DE)
(72) Inventor: Schmiedl, Dieter Dr., 04626 Schmölln (DE)
(74) Representative: Becker Kurig Straus

(57) **Abstract**

Coding for a sample carrier having at least a swab shaft and a piece of cotton wool or the like as sample collector, **characterized in that** said swab shaft (2) has a direct identification (5) in form of a barcodc, a RFID-transponder or another technically accepted labelling, or which swab shaft (2) is alternatively firmly connected to a tube-shaped casing (4) carrying said identification (5).

## Description

### Field of application

The invention pertains to a coding for a sample carrier for securing the identification of a sample. The sample carrier and therewith also the coding according to the invention is a component of an apparatus system for sampling, identifying/labelling, storing, and further processing and providing, respectively, of microbiological, virological, genetical, medical, veterinary medical, forensic, criminalistic and technical samples, wherein a casing carrying the coding takes an important function in identification.

### State of the art

Collecting systems having swabs/cotton wool swabs as actual sample carrier are common in forensics and criminalistics. These swabs mostly consist of a shaft/stick made of wood, metal or plastics to which a piece of cotton wool is provided on one or both ends. A sample can be taken by rubbing off surfaces or wiping. The sample may then be further processed.

Most of the swabs having a long shaft are normally transferred to sealable test-tubes or cardboard boxes.

According to DE 20 2007 001 898 swabs may be inserted into a recess in the closure plug of a sample container.

Yet, the identification of the sample carrier turns out to be problematic. A direct marking/labelling of the container, for example by means of a barcode, a RFID-transponder or a simple inscription is not common and is often not possible because of reasons regarding the apparatuses.

Thus, the sample carriers remain "anonymous" in the sample container until they are further processed.

Hence, the risk of mixing up samples after removal from the sample carrier is quite high.

While the actual sample is kept in a closed and mostly sealed container its identification in form of an inscription, barcode or the like is accessible without a problem, leading to high risks in view of sample safety. The method described above and the technical means available do therefore not meet the requirements of a modem quality management.

The above draw-backs shall be overcome by the present invention described in detail in the following.

### Problem

The problem underlying the present invention derives from the drawbacks of the described state of the art and is to be seen in overcoming said drawbacks.

For this purpose a practical solution will be presented, which comprises an identification/labelling mounted retrofittably and irreversibly to a sample carrier. In this respect the term sample carrier comprises swabs consisting of a swab shaft having a piece of cotton wool mounted on one or both sides. It also encompasses sample carriers which can be inserted into a container seal.

### Solution to the problem

According to the present invention a coding for a sample carrier is provided. The sample carrier has at least a swab shaft and a piece of cotton wool or the like as actual sample collector. The present invention is characterized in that said swab shaft has a direct identification in form of a barcode, a RFID-transponder or another technically accepted labelling. Alternatively, the swab shaft is firmly connected to a tube-shaped casing carrying said identification/label.

In an embodiment said tube shaped casing consists of a firm or flexible material.

In a further embodiment of the present invention the identification is attached to an outside of said casing.

In yet a further embodiment the casing is pushed onto said swab shaft.

In another embodiment of the present invention the tube-shaped casing is slit in a longitudinal direction, wherein effectively functioning slit edges are formed in such manner that said swab shaft can be inserted into the casing.

In yet another embodiment the effectively functioning slit edges of the casing are outwardly bevelled, rounded or curled, or are provided with a bulge, and said effectively functioning slit edges thus formed extend over the complete edge-length of the slit of the casing or, alternatively, comprise one or more portions thereof.

In a further embodiment of the present invention a removal of said casing from said swab shaft is constrained by arresting devices, claws, rough contact surfaces or adhesive areas.

In yet a further embodiment of the present invention the casing is provided with an adhesive depot, which opens upon insertion of the swab shaft.

According to the invention it is proposed to provide commonly known sample carriers used for the described purpose - namely a sample carrier having a swab shaft and a piece of cotton wool arranged on one or both sides - with a permanent direct or indirect identification/labelling, namely a barcode, a transponder or another technically accepted labelling in the region of the swab shaft. A producer may, for example, print or bum the identification/labelling onto the swab shaft or attach the same directly and permanently in a different way.

The indirect labelling is intended for retrofitting established systems. For this purpose a casing made of firm or flexible material is provided with the actual identification/labelling, which casing surrounds the swab shaft. The retrofittable casing is designed in a tube-shaped way and can thus be pushed or slipped onto the swab shaft.

Slipping the casing onto the shaft should only be possible in one direction.

Constricting means shall counteract any pulling of the casing from the shaft, for example by arresting devices, claws, rough contact surfaces or adhesive areas. In cases in which the casing cannot be slipped onto the swab shaft, a longitudinally slit variant is provided. The swab shaft can be inserted into the casing via a slit. The longitudinal edges of the casing are advantageously outwardly bevelled, rounded or curled or are provided with a bulge. The effectively functioning slit edges thus formed must not compulsively extend over the complete edge-length of the slit of the casing, but instead can comprise one or more portions thereof.

Furthermore, the dimensional ratios and technical means are selected so that the casing will firmly and permanently surround the sample carrier, generating an irreversible friction-locked connection between these components.

Moreover, further mounting options are envisioned, namely
- at least one and better more attachment devices, for example claws, are arranged in the interior of the casing. Upon insertion of the swab shaft into the casing they will spread and thus keep the swab shaft in place;
- the inner surface of the casing may contain a surface roughness sufficient for safe adhesion of the casing on the swab shaft;
- the inner surface of the casing can be provided with an adhesive depot, which opens upon insertion of the swab shaft into the casing. A permanent adhesive bond may thus be provided.

Mixing up of samples or a subsequent manipulation is almost excluded and essentially prevented, respectively. The legally relevant issue of securing the sample identification/labelling is complied with by the safe connection of the casing, in slit or non-slit form, with the sample carrier.

A sample labelling according to the invention, which is arranged in a sample container, can be read from the outside without opening the container.

Additionally, a sealed accommodation may protect both, the sample and its label from manipulations.

The sample carrier may be in form of a container seal provided together with a suitable container or tube-shaped casing. The container may be provided with a ventilating hole in order to permit drying of the sample. The ventilating hole may be formed in the wall of the container, preferably near the location where the sample is kept in the container. Preferably, the container is provided with several ventilating holes having a diameter of less than 1 mm. The use of a multitude of small holes reduces the risk of contamination from the environment in comparison with the situation in which one larger hole is used. The one or more ventilation holes may be provided each with respective filter elements. Provision of a ventilating hole with a filter element provides the effect that on the one hand contaminations are excluded but on the other hand effective drying of the sample is still afforded. The filter elements may exhibit a pore size in the range of from 0.1 to 20µm, preferably 0.2µm to 1µm, more preferably 0.2µm to 0.5µm and most preferably approximately 0.22µm. The container may exhibit a length of 10-23 centimetres, preferably 15-18 centimetres, and the container preferably has a diameter of 8-20 mm, preferably 10-15 mm. In many cases the container is substantially made of a transparent plastic, so that a visual inspection of the contents is possible without opening the container.

This container has preferably a top opening for the insertion of a forensic sample or another sample. The top opening may be surrounded by an essentially vertical wall enclosing an inner chamber that is limited in depth by the bottom. The wall and the bottom of the container are usually impermeable to fluids. The container may also comprise a basket and an essentially horizontal intermediate floor for the retention of the sample during washing, i.e., digestion/lysis and extraction. The intermediate floor is permeable to fluids and divides the inner chamber into an upper sample space and a lower fluid space.

The term "forensic" as used herein refers to anything, which has a legal or criminological character. The term is thus not only restricted to the fields of criminal law (e.g., legal medicine), but rather comprises any professional activity within any legal proceeding. Forensically relevant samples also comprise proteins (e.g., the prions causing Creutzfeld-Jacob syndrome, or bovine spongiform encephalopathy, or BSE respectively), viruses, bacteria, and other microorganisms, human or animal bodily fluids (such as blood, sputum, feces, sperm, and urine), and single cells (such as oral mucosa cells and hair follicles). Methods for isolating and analyzing human deoxyribonucleic acid (DNA) ribonucleic acids (RNA) are well known in the art (cf., for example, Molecular Diagnostics: Isolation and Analysis of Human Genomic DNA, 1998 Promega Notes No. 68, p. 20). These methods comprise the PCR methods (PCR = Polymerase Chain Reaction) well known per se for increasing the sample yield and thus the sensitivity of the analysis.

The container seal and/or the container may comprise a removable envelope, for example a blister pack, in which the container seal and/or the container are packaged individually or together. The envelope provides preferably a hermetic seal under sterile conditions, more preferably the envelope's content is DNA free and more preferably the envelope's content is DNA and RNA free.

The container seal and/or the container may be made of any suitable polymer material and manufactured by any suitable method such as injection molding. The preferred polymer material for injection molding of parts of the container seal and/or container is polypropylene.

Labelling provides clear identification of e.g. a particular forensic sample and the biological material extracted therefrom. It is of particular advantage if container seal and container are provided with an identification tag since in this case unambiguous attribution of the sample to container seal and container is possible.

The information or identification tag is advantageously selected from a group comprising a 1-D bar code, a 2-D bar code, an RFID transponder, and a RuBee transceiver. The principle of 1-D and 2-D barcodes is well known to those skilled in the art and is based on the optical scanning of a high-contrast identification marking. The advantage of such identification is the relatively simple physical principle; however, there must be a visual contact between the scanning device and the information tag. RFID transponders are also known per se and operate at high frequency (HF, such as 900 MHz) or ultra-high frequency (UHF). They transmit and receive radio signals, while the newer RuBee transceivers operate at wavelengths below 450 kHz and emit and receive signals, which are primarily based on magnetism. The passive RFID transponders may receive approximately 100 (HF) or 150-200 (UHF) messages per second. In contrast, the active RuBee transceivers may only receive approximately 10 messages/second; visual contact is not needed in any case. The type of information tag used is thus a function, inter alia, of the density of the data transfer and the presence of a visual contact. Any arbitrary combination of bar codes and RFID transponder or a RuBee transceiver are conceivable, this is especially the case when e.g., a simple 1-D bar code is used to identify a sample such as a forensic sample during collection in the field and an RFID transponder or a RuBee transceiver is utilized on the same tube during automatic extraction of the relevant biological material in a high through put workstation of e.g. a forensic laboratory.

The present invention is illustrated by the following example without limiting it thereto.

### Example

In the following example some design options of the coding in form of a casing and of a permanent connection of casing and sample carrier, namely the swab tip, is described with respect to the drawings.

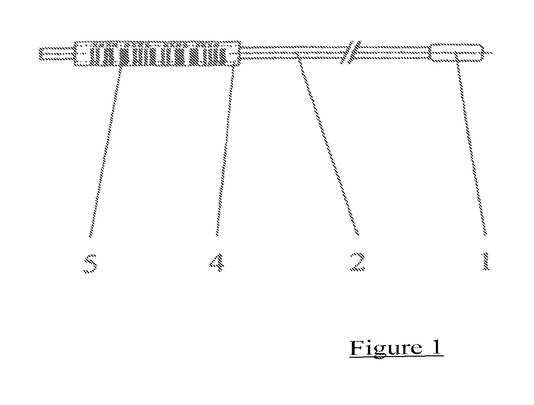

In Fig. 1 a sample carrier in form of a shaft swab is presented, consisting of the swab shaft 1 and a piece of cotton wool 2 attached to one end thereof.

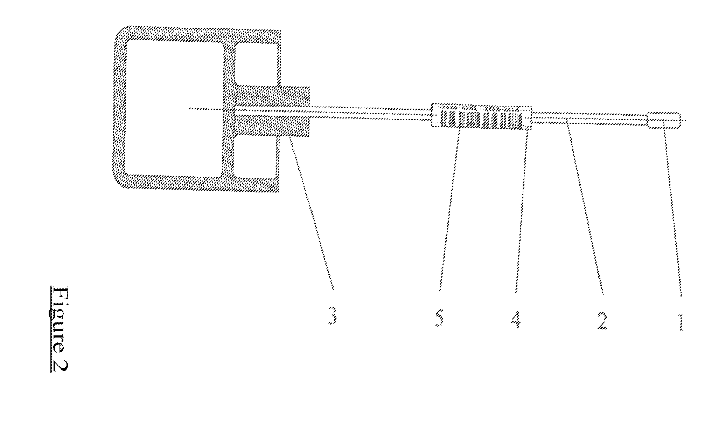

The swab represented in Fig. 2 is connected to the container seal 3 on one side.

Swab shaft 1 is in each case partially surrounded by a casing 4. Said casing 4 selectively carries an identification 5, optionally in form of a barcode, a RFID-transponder or any other technically accepted labelling.

The other figures show embodiments of casing 4.

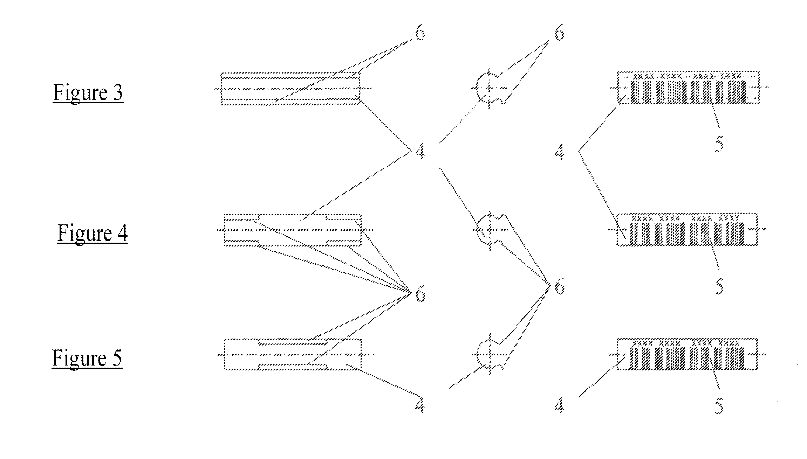

Fig. 3: Casing 4 with continuous effective functioning slit edges 6, in rear, side-and front-view. A barcode is applied to the casing;

Fig. 4: Casing 4 with effective functioning slit edges 6 arranged at the ends, in rear, side and front-view. A barcode is applied to the casing;

Fig. 5: Casing 4 with effective functioning slit edges 6 arranged in the center, in rear, side and front-view. A barcode is applied to the casing.

In order to avoid repetitions it is referred to the above described designs.

### Reference list

- 1: Piece of cotton wool
- 2: Swab shaft
- 3: Container seal
- 4: Casing
- 5: Identification/label
- 6: Effective functioning slit edge.

## Claims

1. Coding for a sample carrier having at least a swab shaft and a piece of cotton wool or the like as sample collector, **characterized in that** said swab shaft (2) has a direct identification (5) in form of a barcode, a RFID-transponder or another technically accepted labelling, or which swab shaft (2) is alternatively firmly connected to a tube-shaped casing (4) carrying said identification (5).

2. The coding for a sample carrier according to claim 1, **characterized in that** said casing (4) consists of firm or flexible material.

3. The coding for a sample carrier according to claim 1, **characterized in that** said identification (5) is attached to an outside of said casing (4).

4. Coding for a sample carrier according to claim 1, **characterized in that** said casing (4) is pushed onto said swab shaft (2).

5. Coding for a sample carrier according to claim 1, **characterized in that** said tube-shaped casing (4) is slit in longitudinal direction, wherein effectively functioning slit edges (6) are formed in such manner that said swab shaft (2) can be inserted into said casing (4).

6. Coding for a sample carrier according to claim 5, **characterized in that** said effectively functioning slit edges (6) of casing (4) are outwardly bevelled, rounded or curled or are provided with a bulge, and that said effectively functioning slit edges (6) thus formed extend over the complete edge-length of the slit of the casing (4) or, alternatively, comprise one or more portions thereof.

7. Coding for a sample carrier according to claim 1, **characterized in that** the removal of said casing (4) from said swab shaft (2) is constrained by arresting devices, claws, rough contact surfaces or adhesive areas.

8. Coding for a sample carrier according to claim 7, **characterized in that** said casing (4) is provided with an adhesive depot, which opens upon insertion of the swab shaft (2).
